# EUROPEAN PATENT APPLICATION

(11) **EP 3 698 773 A1**
(43) Date of publication of application: **26.08.2020**
(21) Application number: 19425007.2
(22) Date of filing: 21.02.2019
(51) Int. Cl.: A61K 9/00, A61K 39/00, A61K 47/02, A61K 47/14, A61K 47/26, A61K 47/36, A61K 9/107, A61K 9/16

(54) **COMPOSITION AND MANUFACTURING OF POWDERS CONTAINING NANOADJUVANTS FOR MUCOSAL VACCINATION**

(71) Applicant: Università degli Studi di Parma, 43121 Parma (IT)
(72) Inventor: Sonvico, Fabio, I-43126 Parma (IT); Bettini, Ruggero, I-43035 Felino (PR) (IT); Martelli, Paolo, I-43125 Parma (IT); Borghetti, Paolo, I-43029 Traversetolo (PR) (IT); Ferrari, Luca, I-43123 Parma (IT); Canelli, Elena, I-42030 Viano (RE) (IT)
(74) Representative: Gerli, Paolo

(57) **Abstract**

New preparative approach of dry powder vaccines for mucosal (e.g. nasal) administration for the purpose of human or animal immunization; it requires spraying a vaccine liquid dispersion, previously mixed with a sub-micron particulate adjuvant, onto a solid carrier while blending the mixture, followed by drying in mild conditions; the sub-micron particulate adjuvant is an O/W nanoemulsion stabilized with a polysaccharide. Improved dry powder vaccines are obtained in form of aggregated antigen-carrier particles, whereby the antigen is finely and firmly dispersed within the carrier; once in contact with the mucosal surface, the product quickly dissociates and releases the antigen component.

## Description

### FIELD OF THE INVENTION

The present invention is generally related to vaccines for prevention of infectious diseases and more specifically related to vaccine in powder form to be administered nasally or, more generally, to any mucosal tissue.

### BACKGROUND OF THE INVENTION

Traditionally, most vaccines have been delivered parenterally using liquid formulations.

Independently from the type of vaccine considered, i.e. live attenuated, killed inactivated or subunit vaccines, antigens are known to be prone to degradation or modifications in aqueous conditions, which require a specific formulative approach, packaging and storage conditions, such as a cold chain, to provide stability and consequently ensure safety and efficacy. In addition, parenteral administration requires sterile products and devices, along with administration by trained healthcare professionals¹.

The use of alternative routes of vaccination *via* the mucosal routes could potentially provide a convenient, safe, non-invasive and needle-free option for vaccination.

Mucosal surfaces are a major portal of entry for many pathogens and for this reason a specialized interconnected mucosal immune system provides constant immune surveillance. This network of immunocompetent tissues as a consequence can be used as a route for vaccination. Mucosal vaccine-induced immune responses are initiated in specific mucosa-associated lymphoid tissue (MALT) structures²

Within the possibilities (options?) for mucosal vaccination, nasal administration is one of the most promising routes due to reduced enzymatic activity, a relatively permeable epithelium, and the presence of highly available immuno-reactive sites³.

In particular, nasal associated lymphoid tissue (NALT), that include the so-called Waldeyer's ring composed of: the nasopharyngeal tonsil or adenoid (NT), the pair of palatine tonsils (PT), the pair of tubal tonsils (TT) and the lingual tonsils (LT), appears as a remarkable target for vaccination strategies⁴. Interestingly, a similar immunocompetent ring is also present in pigs, which makes this animal a good model for nasal vaccination or a potential target for veterinary vaccination.

Vaccines for mucosal vaccination via the respiratory tract have been already developed and marketed: the influenza vaccine FluMist® (Medimmune/Astra Zeneca, UK) based on a cold-adapted influenza strain is available in US and Canada⁵, while the live attenuated H1N1 influenza vaccine NasoVac® (Serum Institute of India Ltd, India) was developed in 2010 at the peak of the pandemic alert and administered to more than 2.5 million people⁶. Both vaccines are administered as liquid nasal spray.

Formulation strategies adopted in recent years to obtain robust immune responses have been based obviously on the selection of strongly immunogenic antigens but also on their formulation with additional components called adjuvants able to direct and enhance the immune response. Adjuvants are often particulate carriers and/or immunostimulatory substances, and it has been demonstrated that those in the nanometer size range can be used to protect the antigen, prolong mucosal residence, target the mucosal immune system increasing uptake by M-cells for subsequent delivery to antigen presenting cells and stimulate both innate as well as adaptive immune responses⁷.

Among adjuvants, the most commonly used are mineral salts (aluminium salts), tensioactives (Quil A derived saponins), bacteria-derived toxins and immunostimulatory substances (Cholera toxin, heat-labile enterotoxin, monophosphoril lipid A, muramyl dipeptide, threalose dimycolate), emulsions (both O/W or W/O, such as Freund's ajuvant, Montanide), cytokines (IL-12), nucleic acids-based adjuvants (CPG-ODN) and polysaccharides (chitosan)⁸. However, it has been demonstrated that particulate antigen delivery systems contribute to improve the efficiency of mucosal vaccines, with M-cells able to ingest microparticles with diameters between 1 and 5 µm, while nano-sized particles can be endocytosed by epithelial cells⁷, leading to the use of polymer (poly(lactide), poly(lactide-co-glycolide), poly(ε-caprolactone), polyethyleneimine) or polysaccharide (inulin, chitosan)-based micro- and nanoparticles, liposomes (cationic, pH sensitive, cell-penetrating), micelles (poloxamers) and nanoemulsions (MF59, W₂₀5EC and W₈₀5EC)^{3,8,9}.

A paradigmatic example of nano-sized particulate systems combining several of the adjuvant substances mentioned are immune-stimulating complexes, also called ISCOMs. ISCOMs are 40 nm vector particles containing QuilA saponins, cholesterol and phospholipids that can be associated with antigens directly or added to an existing formulation to potentiate the immune response. Helgeby and co-workers demonstrated that after nasal administration, a cholera toxin A1/ISCOMs combined vector is a highly effective enhancer of a broad range of antibody and cell-mediated immune responses against a model antigen from influenza virus¹⁰.

In the latter case, as well as in many of the vaccines developed for mucosal delivery, the formulation is administered in liquid form. The use of dry powder formulations appears promising to provide physical, chemical and microbiological stability to the formulation, potentially avoiding the need for preservatives, buffers and cold-chain storage and transportation¹¹.

At the same time, the use of a dry powder formulation represents a challenge for the inclusion of nanometric-sized vectors and adjuvants since, because of their size and composition, they might be prone to aggregation³ and commonly adopted drying processes are known to affect the size of sub-micron particles, such as the mentioned vaccine vectors, adjuvants and antigens¹²⁻¹⁴.

Huang et al. proposed a dry powder nasal vaccine for influenza prepared by freeze-drying of whole inactivated influenza virus followed by milling and sieving and containing chitosan as mucoadhesive¹⁵. However, the IgA responses elicited *in vivo* were variable as a consequence of powder broad particle size distribution and morphology and the same group proposed spray-freeze-drying as an alternative process. The authors demonstrated that among different mucoadhesive adjuvants, sodium alginate-containing powders provided greater stability compared to liquid formulation, increased residence time in the nasal cavity and IgG and IgA responses comparable with an inactivated whole influenza virus liquid formulation administered intranasally (IN) or intramuscularly (IM)¹⁶.

Spray freeze-drying was also employed to produce anthrax vaccine powder formulations containing recombinant protective antigen of Bacillus anthracis combined with a CpG-containing oligonucleotide¹⁷ or a mast cell activator compound¹⁸ as adjuvant in view of a nasal mucosal delivery. Powder formulations offered an improved storage stability compared to the liquid formulation and comparable toxin neutralizing antibodies to an intramuscular immunization with the same antigen.

On the other hand, freeze-drying and milling have been successfully applied to the development of proprietary GelVac™ vaccine powders for the nasal immunization against viral gastroenteritis, the formulation and use of which are described in the patent US9439958B2 and patent applications US20140286994A1 and WO2017040265A1.

The formulation contains recombinant norvirus virus like particles (VLPs) and a mucoadhesive anionic polysaccharide derived from *Aloe vera* (GelSite®) and it was shown to induce systemic and mucosal neutralizing antibodies of each of the VLPs in a dose-dependent manner after intranasal administration in guinea pigs, even without traditional adjuvants^{19,20}.

The freeze-drying process involves a number of physical stresses such as freezing (concentration of particulates and/or crystallization of solutes), sublimation and desorption of (dehydration) that could affect antigen and sub-micron particulate vectors/adjuvant stability²¹. Li and coworkers demonstrated that thin-film freeze-drying, a technique that consist in drying a frozen film by lyophilization, was able to convert liquid vaccines adjuvanted with aluminum salts into dry powders without causing particle aggregation or decrease immunogenicity of the vaccines²². The Chinese patent application CN105342982 reports a nasal vaccine composition for influenza, containing an osmotic pressure regulator, a pH adjuster and an immunopotentiator selected among alkyl glycosides, chitosan, low acyl gellan or liposomes and reports also the possibility to lyophilize the preparation in presence of mannitol, sucrose, lactose or other lyoprotectants for its use as a dry powder vaccine.

An alternative to freeze-drying is the use of spray-drying techniques. Spray-drying provides a rapid evaporation of the aqueous vehicle, avoids freezing and high vacuum, is a continuous process with lower operating costs and allows for the production of a fine powder avoiding the necessity of a milling step²³.

The same group that developed the vaccine for viral gastroenteritis already cited, used spray-drying as an alternative to freeze-drying to produce a dry powder containing Norwalk virus-like particles (NV VLPs) and the inert *in situ* gelling polysaccharide (GelSite) extracted from Aloe vera for nasal delivery and showed in guinea pig that the powder formulation was able to delay mucociliary clearance and thereby and prolong VLP antigen exposure to immune effector sites²⁴.

Jones et al. produced by spray-drying a second-generation anthrax vaccine formulations based on plant-produced, deglycosylated recombinant protective antigen (PA), trehalose, hydrolyzed gelatin and the adjuvant Alhydrogel. All formulations resulted stable even under 3 months of accelerated stability storage conditions (40°C/75%RH) and retained their immunogenic properties¹⁷.

Muttil and his group developed a dry powder for human papilloma virus (HPV) using a bacteriophage virus-like particle associated with excipients such as D-mannitol, L-leucine, D-(+)-trehalose and dextran by optimizing the drying parameters using a half-factorial design approach. The prepared powders were stable after 12 months of storage at 37°C and maintained immunogenicity after enteric or intramuscular immunization²⁵.

Spray-drying is claimed in the patent application EP1834651A1 as the preferred method to obtain a powder composition with average particle size from 2 to 30 µm and narrow size distribution for veterinary vaccination by inhalation against avian viral infections.

Yet also spray-drying has been shown to present some drawbacks: antigen and particulate vector/adjuvant are exposed to shear stress, elevated temperatures and formation of air-water interfaces during the formation process may lead to antigen denaturation, particle aggregation and reduction of vaccine efficacy. Furthermore, an additional drying step could be required to obtain very low residual water content in the end product.

The already cited spray freeze-drying, combining both processes eventually sum up the physical stresses to which the formulation is subjected instead of reducing them. However, the patent application EP1972347A1 disclosed a method for preparing stable vaccine powder formulations in which spray-freeze drying or atmospheric spray-freeze-drying are used to prepare particles containing antigen, excipients selected among mannitol, trehalose, dextran and an adjuvant such as lipopolysaccharide for further reconstitution in liquid form in view of immunization by minimally invasive administration techniques.

The use of pharmaceutical solid excipients as carriers for a dry powder vaccine formulation is *per se* known. In patent application EP2689785A1 (related to the international applications PCT/JP2011/002225 and WO2011/129120) is a disclosed method for the preparation of a dry powder influenza vaccine through a quick freezing step followed by freeze-drying overcoming the limitations of traditional lyophilization processes. The formulation described comprise one or more antigens, such as whole inactivated influenza virus, and one or more excipients, such as a saccharide (trehalose, mannitol or lactose) or a buffer (phosphate). In view of nasal administration, the obtained powder can be blended with a suitable nasal carrier or flowability agent such as for example microcrystalline cellulose and tribasic calcium phosphate.

Preformed bioadhesive spray dried particles of starch (Amioca®) and crosslinked poly(acrylic acid) (Carbopol® 974P), disclosed in the patent application US20030143277A1, were used as carriers of heat-inactivated influenza virus and heat-labile enterotoxin adjuvant and transformed in a powder vaccine by freeze-drying in view of nasal administration by Coucke and co-workers²⁶. Trows and Scherließ recently proposed to mix antigen-loaded chitosan microparticles with carrier materials such as sugar alcohols (mannitol, sorbitol, maltitol) by mechanical blending in order to improve nasal deposition of nasal dry powder vaccine²⁷. The previous approach is further detailed in the patent application US20170143822A1, where antigen-loaded chitosan nanoparticles are embedded by spray-drying in microparticles in which the matrix agent is a monosaccharide, a disaccharide, a sugar alcohol or a polysaccharide and then this material may be admixed to carrier particles in order to improve flow and loading in dry powder inhalers.

In another approach disclosed in the patent applications US2011159047A1, US20080226729A1 and related applications (EP2068834A2, JP2010502747A and WO2008079464A8), the dry powder vaccine is obtained by spray-freeze-drying or atmospheric spray-freeze-drying by adsorbing the antigen onto aluminium adjuvants in presence of at least one excipient among mannitol, trehalose and dextran.

In the patent application US20120045479A1, respirable dry powder HPV vaccine formulations were obtained by drying via carbon dioxide assisted nebulization (CAN) a solution of HLP L1 capsid protein and myo-inositol and leucine, used as carriers.

In the US patent 5,942,242 the inventors claimed the use of cation exchange resins and salts thereof, selected among polystyrene-sulfonates, polystyrene-acid copolymers and with particle size not larger than 200 µm, as a carrier for peptides and proteins to be used therapeutically or for vaccination by nasal delivery. In the preferred embodiment, the powder is prepared by mixing carrier and a vaccine liquid preparation to form a suspension that is then freeze-dried and ball milled.

In the Japanese patent applications JP2009209086 and JP2011057605 a vaccine for mucosal application is disclosed comprising a pathogen-derived antigen, an adjuvant, indicated as a fine powder of double-stranded or single-stranded RNA which is a ligand of Toll-Like Receptor or chitin or chitosan and a thickening agent selected among alginates, cellulose or starch derivatives, and polyacrylates. It is claimed that the preparation can be conveniently administered on the mucosal tissues as a powder.

The patent US6391318B1 discloses a nasal vaccine composition that utilizes the cationic polysaccharide, chitosan, with different molecular weights (50 kDa-2,000 kDa) and degree of acetylation (40%-98%) as a delivery system. The intranasal compositions according to the invention can be formulated as liquids or dry powders, for administration as aerosols, drops or insufflations. Preferably, the compositions according to the invention are formulated as dry powders or in the form of microspheres.

The Chinese patent CN104208029 discloses a dry powder vaccine composition for nasal administration comprising an antigen (influenza virus antigen, tetanus antigen and hepatitis B virus antigens), an oil phase (squalene, squalane, medium chain triglycerides, fish oil, olive oil and soybean oil), one or more surfactants (polysorbate, polyethylene glycol 12-hydroxy stearate polyethyleneglycol-12-hydroxy acid ester and a mixture of alcohol and a mixture of phospholipids), a mucoadhesive membrane (chitosan, cellulose derivatives, sodium alginate, carbomer), a freeze-drying excipient (lactose, sucrose, trehalose, amino acids, mannitol) and a buffer (phosphate, 4-hydroxyethyl piperazine ethane sulfonic acid and citric acid) transformed into a powder by freeze-drying and subsequently is physically mixed with a carrier.

Despite the evolution of research on powder antigen formulations, it remains difficult to provide products meeting different goals like e.g.: ultra-fine, homogeneous and firm dispersion of the antigen in a pharmaceutical carrier (thus being optimally conveyable to a patient); quick dissociation upon contact with body liquids (thus quickly releasing the antigenic component); very mild production conditions (thus minimizing antigen degradation and ensuring a high immunogenic potency).

### SUMMARY OF THE INVENTION

The invention discloses an improved preparative approach and the resulting aggregated composition for dry powder vaccines, useful for the nasal or, more generally, mucosal administration in view of human or animal immunization.

The solution envisaged in this patent is characterized by spraying a vaccine liquid dispersion, mixed with a "sub-micron particulate adjuvant", onto a solid carrier while blending the mixture, followed by drying in mild conditions. The sub-micron particulate adjuvant is an O/W nanoemulsion stabilized with a polysaccharide; the solid carrier can be selected among soluble and insoluble pharmaceutical excipients useful for this purpose. In a non-limitative preferred embodiment: the adjuvant comprises alpha tocopherol and/or sunflower oil as oil phase, in association with PEG 660 12-hydroxystearate as a surfactant and with low molecular weight chitosan as a stabilizing polysaccharide; further in this embodiment, the solid carrier is calcium carbonate, and the antigen is an inactivated whole-cell concentrate of *Mycoplasma hyopneumoniae.* The products obtained by the present invention are physically characterized as aggregated antigen-carrier particles, whereby the antigen is finely and firmly dispersed within the carrier; this result is advantageously achieved without recourse to high-impact compounding methods (i.e. high energy mixing or use of elevated temperatures) which may reduce the antigen activity; furthermore, despite the firm antigen-carrier aggregation, the product quickly dissociates and releases the antigen component when reconstituted in an aqueous environment, such as on a mucosal surface, allowing a quick release of the particulate vaccine components.

### DESCRIPTION OF THE FIGURES

FIG. 1: Shows the dependence of average size and PDI (polydispersity index) of NE (nanoemulsion) containing PEG 660 12-hydroxystearate on the surfactant to oil ratio (SOR) present in the composition.
FIG. 2: Shows the dependence of average size and PDI of NE containing PEG 660 12-hydroxystearate on the surfactant to oil ratio (SOR) present in the composition.
FIG. 3: Shows the dependence of average size and PDI of NE containing PEG 660 12-hydroxystearate on the surfactant to oil ratio (SOR) present in the composition.
FIG. 4: Shows SEM pictures of nanoemulsion dried on mannitol (A) and calcium carbonate (B) at magnification factor 400X (A1, B1); 3.00 KX (A2, B2) and 5.00 KX (A3, B3).
FIG. 5: Shows the average size of particles obtained after redispersion in water of dry powders obtained by drying O/W nanoemulsions (NE) on different solid carriers: mannitol (Man, white bars), calcium carbonate (CaC, dark bars) and their 1:1 mixture (M/C, grey bars) at different liquid to solid weight ratios: 1:1, 2:3, 1:2.
FIG. 6: Shows the polydispersity index of particles obtained after redispersion in water of dry powders obtained by drying O/W nanoemulsions (NE) on different solid carriers: mannitol (Man, white bars), calcium carbonate (CaC, dark bars) and their 1:1 mixture (M/C, grey bars) at different liquid to solid weight ratios: 1:1, 2:3, 1:2.
FIG. 7: Shows the particle size distribution by volume (solid line) and cumulative undersize curve (dotted line) of inactivated whole-cell concentrate Mycoplasma hyopneumoniae.
FIG. 8: Shows the particle size distribution by volume (solid line) and cumulative undersize curve (dotted line) obtained after the redispersion in water of the dry powder vaccine produced drying a mixture of O/W nanoemulsion and inactivate whole-cell concentrate Mycoplasma hyopneumoniae (40:60) on mannitol solid carrier (proportion liquid to solid solid to liquid ratio 2:1).
FIG. 9: Shows porcine nasal mucosa at 2, 5 and 9 days post-vaccination with a dry powder vaccine administered nasally. Circles shows cells with phagocytic activity.
FIG. 10: Immunohistochemical staining on a porcine nasal mucosa section showing cells that express SLA Class II antigen.
FIG. 11: Shows the cellular immune response in terms of frequencies of Mhyo-specific IFN-γ secreting cells in PBMC in intramuscularly (IM) and intranasally (IN) vaccinated pigs.
FIG. 12: Shows the cellular immune response in IM- and IN-vaccinated pigs in terms of percentages and stimulation indexes (SI) of Mhyo-specific CD79α+IgA+ and CD79α+IgG+ memory B cells in PBMC after in vitro re-stimulation with the Mhyo antigen.

### DETAILED DESCRIPTION OF THE INVENTION

In the present text, with "dry powder vaccine" it is meant a product, preferably in dosage form, constituted of solid particles that allows for the delivery and the preservation of the functional characteristics of an antigen and a suitable adjuvant, in order to elicit in a human or an animal an effective immune response.

The terms "aggregate" or "aggregated formulation" identify a solid product comprising particles of different substances which (differently from a physical mixture of the same) are aggregated together in larger agglomerates with particle size comprised between 0.5 and 500 µm In said agglomerates, the aggregated original particles remain recognizable, e.g. at microscope observation; also differently from physical mixtures, in the present "aggregate", free, non-aggregated particles can be present only at traces level, typically less than 20%, or even less than 10% by weight of the product as a whole.

The expression "sub-micron", referred to the present particulate adjuvant, means nanometre-sized (size below 1000 nm, preferably below 200 nm) solid particles or droplets; the same particle size is meant herein for the term "O/W (oil-in-water) nanoemulsion", wherein the nanoemulsion is obtained with the aid of one or more surfactants of an oil or a mixture of oils in an aqueous continuous phase.

With "solid carrier" it is meant a pharmaceutical dry powder excipient, water soluble or insoluble, with particle size suitable for mucosal administration, typically in the range 10 to 200 µm, preferably below 110 µm.

With "spraying" or "dispersing as a spray" is meant any technical mean of distribution of a liquid onto a solid in form of droplets with particle size between 1 and 5000 µm ; such spraying is suitably aided by means to control the volume and rate of addition.

With "blending" is meant any technical mean of mixing powders and liquids.

With "drying in mild conditions" is meant any technique of solvent evaporation avoiding high temperatures or high vacuum, i.e. applying temperatures typically in the range 0 to 60°C, preferably from 0 to 50°C, and/or applying pressure lesser than 1 bar, e.g. from 1 mbar to 800 mbar, and avoiding freezing as a step to obtain water or aqueous media sublimation.

With "release of the particulate vaccine components" it is meant the total or prevalent reconstitution, starting from the dry powder formulation, of the original components after dispersion in an aqueous environment.

With "effective immune response" is meant an immune response in terms of humoral and/or cellular reactivity/positive modulation at least equivalent to the one elicited by a control intramuscular administration of the same antigen.

An object of the present invention is a process to obtain a highly-active antigen aggregated formulation for nasal or, more generally, mucosal administration, comprising the steps of:
(a) adding a liquid dispersion of the antigen to an oil-in-water nanoemulsion, in the respective vol/vol ratio from 10:1 to 1:1; wherein said oil-in-water nanoemulsion comprises a surfactant and an oligosaccharide or a polysaccharide and has a surfactant/oil weight ratio (SOR) ranging from 20% to 80%;
(b) dispersing as a spray the product of step (a) onto a solid carrier in movement, wherein said product of step (a) and said carrier are used in the respective wt/wt ratio from 0.1:1 and 4:1, and subsequently drying the resulting mixture in mild conditions;

The oil-in-water nanoemulsion mentioned step (a) is also referred herein as "sub-micron particulate adjuvant composition". It contains an oily phase and a water phase; the oily phase contains hydrophobic substances of natural or synthetic origin, in particular those having shown in the known art adjuvant properties, i.e. enhancement of the ability of the vaccine to induce protection against an infection; example thereof are: alpha-tocopherol, squalene, squalane, short or medium chain triglycerides, fish oil, olive oil, castor oil, sunflower oil and the like. Generally, the oil phase in the nanoemulsion is a mixture of pharmaceutical-grade oils, preferably an 1:1 mixture of alpha-tocopherol and sunflower oil. Preferably, said nanoemulsion has a water phase and an oil phase in the respective volume ratio from 1:1 to 6:1. In more preferred embodiments, the nanoemulsion comprises about 5 to 30% w/w, and preferably about 20% w/w of oil phase.

The nanoemulsion also contains surfactants, i.e. amphiphilic substances with both a hydrophilic and a hydrophobic moiety, adapted for the preparation of oil-in-water emulsions. In the preferred embodiments, the nanoemulsion comprises about 5 to 30% w/w, and preferably about 25% w/w of one or more surfactants. The surfactants are prepared in the oil phase and help obtain droplets with submicron dimensions; an important feature of the nanoemulsion is the surfactant to oil weight percent ratio (SOR): is was in fact experimentally found that within SOR values from 20 to 80%, preferably from 50 to 80%, it is possible to obtain a nanoemulsion with the desired particle size, typically below 300 nm. Generally, non-ionic polyethoxylated surfactants with more hydrophilic properties are chosen from, but not limited to: polysorbates, ploxamers, polyoxyethylene alkyl ethers, polyoxyethylene castor oil derivatives, polyoxylglycerides, polyoxyethylene stearates, D-alpha-tocopherol polyethylene glycol succinate (TPGS), polyoxyl 15 hydroxystearate. Thus, in certain preferred embodiments, the compositions of the present invention comprise D-alpha-tocopherol polyethylene glycol succinate (TPGS), polyoxyethylene castor oil or polyoxyl 15 hydroxystearate in proportion to the oil phase ranging from 50:50 to 70:30.

The polysaccharide or oligosaccharide contained in the nanoemulsion is normally present in dissolved form in its aqueous phase; it works as stabilizer of the nanoemulsion, as mucoadhesive and as an adjuvant of the dry powder vaccine. Examples of polysaccharides or oligosaccharides that can be used are chitosan, hyaluronate (preferably with molecular weight in the range from 5 to 250 kDa), starch, inulin, dextrans, maltodextrins, cyclodextrins. Preferably, non-reducing poly/oligosaccharides are used, particularly the cationic polysaccharide chitosan. The latter is preferably used in the aqueous phase of the O/W nanoemulsion concentration between 1 and 10 mg/ml, more preferably at 5 mg/ml concentration dissolved in acetic acid 0.5% v/v aqueous solution. The molecular weight of the chitosan is generally in the range from 10 to 150 kDa and its deacetylation degree above 80%, preferably the molecular weight is below 50 kDa and deacetylation degree above 95%. The use of mucoadhesive polysaccharides provides an advantage to the formulation because of the stabilization effect on the nanoemulsion, furthermore they contribute to prevent the physical alterations of the particulate adjuvant and antigen during the drying process, favouring their release after reconstitution.

The mixing of the antigen liquid dispersion to the sub-micron particulate adjuvant is performed in the respective vol/vol ratio from 10:1 to 1:1, preferably from 2:1 to 1:1.

In step (b) of the present process, the oil-in-water nanoemulsion (sub-micron particulate adjuvant), after being mixed with the antigen liquid dispersion, is brought in contact with the solid carrier by spraying it on the solid carrier kept in movement at a suitable liquid to solid ratio: this is the weight/weight ratio between the amount of sprayed dispersion (as resulting from step (a)) and the amount of solid carrier; this ratio is from 0.1:1 to 4:1, preferably from 1:1 to 3:1. The chosen liquid to solid ratio is such that the liquid sprayed is absorbed by the solid carrier, which maintains its solid state (i.e. it does not form a solution) throughout the whole step b).

The solid carrier is selected among conventional ones, i.e. water soluble pharmaceutical dry powder excipients, such as mannitol, sorbitol, dextrose, sucrose, lactose, water soluble cellulose derivatives and maltodextrin or among insoluble pharmaceutical dry powder excipients, such as calcium carbonate, calcium phosphate salts, microcrystalline cellulose, ethyl cellulose. Preferably, the solid carrier has particles with dimensions ranging from 10 to 200 µm, preferably from 10 to 110 µm. Preferably, the carrier is mannitol or calcium carbonate and it is mixed with the antigen and sub-micron particulate adjuvant mixture (as resulting from step (a)) in a liquid to solid ratio from 1:1 to 2:1.

During spraying, the solid carrier is suitably maintained in movement conditions, e.g. it is agitated, vortexed, kneaded, shaken, etc., thus favouring the homogeneous distribution and layering of the spayed liquid throughout the whole solid carrier used; to obtain these aims, low-energy movement conditions (e.g. less than 10 rpm) are sufficient in the frame of the present invention and are preferably applied in order to further preserve the antigen integrity and activity.

An important feature of the invention lies in that the drying phase is started only after the end of the spraying phase: this separation, distinguishing the present step (b) from conventional spray-drying processes, where the two processes run in parallel, is highly advantageous in that it prevents a premature drying of the sprayed droplets, i.e. prior to their contacting the carrier surface; this enhances the amount of liquid phase (thus of antigen) actually layered onto the carrier, while eliminating or reducing to traces the amount liquid phase being dried in unbound state, i.e. not aggregated to the carrier; as a further advantage, the carrier is maintained in the wetted state, thus being protected against any undesirable temperature increase of the solid mass caused by the applied mixing force. To finally safeguard the antigen activity, the drying phase is performed in mild conditions, in particular under mild heating (typically between 30 and 50°C) and/or by applying mild vacuum conditions (pressure above 1 mbar).

In a variant comprised by the present invention, the spraying step b) can be subdivided a chosen number *n* of cycles: in this case, in each spraying step, only a fraction of the whole amount of liquid resulting from step a) is sprayed; then after interruption of spraying, the carrier is dried according to c); then the procedure is repeated for the *n* number of chosen cycles until the whole amount of liquid has been sprayed; preferably, *n* is 2, 3, 4 or 5. This variant is particularly useful when spraying high volumes of liquid onto the solid carrier.

A further object of the invention is the product as such (dry powder vaccine) as such, as obtained/obtainable by the above described process of mixing the sub-micron particulate adjuvant and an antigen liquid dispersion, followed by the spraying onto a solid carrier, and further followed by drying in mild conditions. The so-obtained product will be characterized by comprising all the ingredients mentioned during the above described preparation process and their mutual ratios, with exception of the water component, which evaporates during the drying phase. The so resulting solid product is further characterized by its aggregated physical nature: in particular, it comprises particles of the different solid substances mentioned above which, differently from a physical mixture thereof, are not free but are aggregated in larger agglomerates (with particle size comprised between 1 and 500 µm.); within these agglomerates, the original particles are still distinguishable, e.g. at microscope observation, as shown in the experimental section. As further shown in the experimental section, this aggregate product is also characterized by its capacity to readily dissociate after contact with water, such as happens in-vivo when the dry powder vaccine comes in contact with the wet nasal or other mucosa. A further feature of the resulting product is the high degree of preservation of the immunizing potency of the original antigen: in particular, the very mild conditions applied throughout the entire production process prevent any undesired degradation of the antigen and reduction of its original potency. Particularly interesting results in this respect are reported in the experimental section, where the immunostimulating potency of the present vaccines, intranasally administered, was found to be robust and even equivalent to conventional intramuscular (i.e. with direct access to the bloodstream, with no need to permeate through mucosa) immunization with the same antigen.

A further object of the invention are pharmaceutical compositions wherein the product of the present invention (dry powder vaccine) is admixed with further pharmaceutically acceptable excipients.

A further object of the invention is the use of the above described product of the invention (dry powder vaccine) and/or its relevant pharmaceutical compositions in a method of immunostimulation of a subject (human or animal) in need thereof. The method is performed via administration of the vaccine on any mucosal surface, preferably the nasal one (nasal administration).

By way of non-limitative example, some preparation examples are reported in the following section.

### EXAMPLES

A dry powder vaccine formulation was developed for the nasal administration in pigs of a vaccine against the enzootic pneumonia resulting from infection by *Mycoplasma hyopneumoniae.* The dry powder formulation included the antigen in form of an inactivated whole-cell concentrate of *Mycoplasma hyopneumoniae,* a sub-micron particulate adjuvant in form of a nanoemulsion prepared with alpha-tocopherol, sunflower oil, PEG 660 12-hydroxystearate as surfactant and low molecular weight chitosan as stabilizer and finally, calcium carbonate as solid carrier.

Such formulation was used as mucosal vaccine in piglets and furthermore, control comparative tests were performed using a reference vaccine formulation administered intramuscularly.

In a first series of experiments, in order to evaluate the characteristics of the dry powder formulation proposed, namely the size distribution of the nanoemulsion and the possibility of reconstituting it after drying, nanoemulsions were produced and then dried on solid carriers in form of powder. In all cases conditions for the production of nanoemulsions with dimensions below 300 nm were identified and then dried on a solid carrier, verifying the possibility of reconstituting the nanoemulsion after dispersion.

In a second series of experiments, it was evaluated the effectiveness of the composition object of this patent application in eliciting a local immune response by evaluating the nasal mucosa histology and immunohistochemistry after nasal administration of the dry powder vaccine in pigs. By comparing a commercial *Mycoplasma hyopneumoniae* vaccine administered intramuscularly to a positive control group of animals, it was possible to evaluate the efficacy of the composition object of this patent application in stimulating specific systemic cellular immune responses against the antigen in terms of frequencies of *Mycoplasma hyopneumoniae*-specific IFN-γ secreting cells and a Mycoplasma hyopneumoniae-specific memory B cells (CD79α+) expressing surface IgA or IgG within peripheral blood mononuclear cells.

### Example 1

The purpose of this example was to describe the preparation of a nanoemulsion (NE) to be used in a *Mycoplasma hyopneumoniae* nasal dry powder vaccine.

In these preparations the aqueous phase and the oily phase were prepared separately. The ratio between the aqueous and oily phase was kept constant at 4:1.

The aqueous phase in this example was a low molecular weight chitosan solution. The 0.5% w/v chitosan solution (pH = 4) was prepared by dissolving 0.5 g of chitosan polymer (MW 30 kDa, deacetylation degree 99%) in water containing 0.5% w/w acetic acid. This solution was then continuously stirred until complete dissolution of the polymer.

The oily phase was composed of alpha tocopherol and sunflower oil in proportion 1:1, mixed at different ratios (surfactant/oil ratio, SOR) with one of three selected nonionic surfactants, i.e. PEG 660 12-hydroxystearate, polyoxyl 35 castor oil and TPGS. The oily phase was prepared adding to the sunflower oil, the viscous alpha-tocopherol and the solid nonionic surfactant, heating at 70°C and stirring until a solution is obtained.

The oily phase was then slowly added into the aqueous phase under vigorous mechanical stirring obtained with an homogenizer, disperser or emulsifier apparatus until a homogeneous non-viscous translucent to white nanoemulsion was obtained.

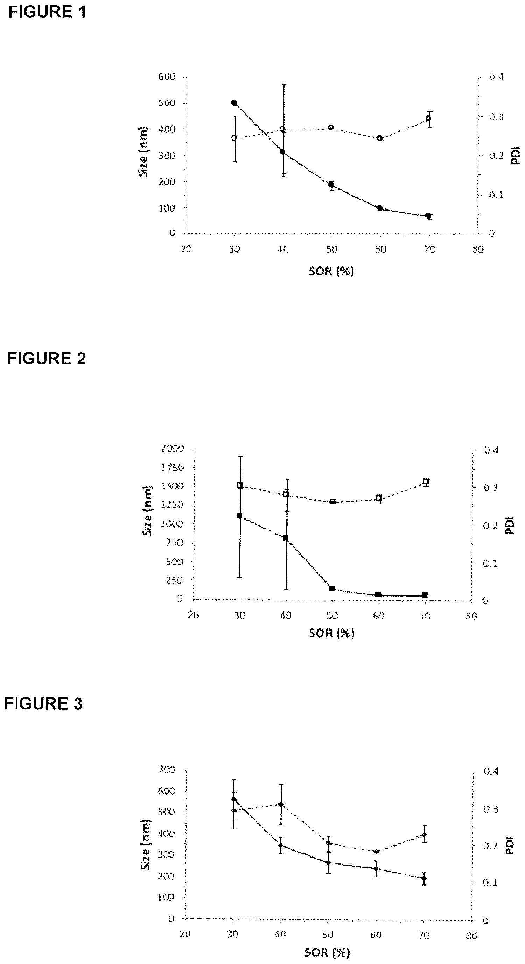

Average particle size and polydispersity index by dynamic light scattering (Zetasizer Nano ZS, Malvern Pananalytical, Malvern, UK) obtained for different SOR in the composition of the nanoemulsion and for the three different surfactants are presented in FIG.1-3.

As it can be appreciated from FIG. 1 to 3 for surfactant/oil ratios (SOR) above the proportion 1:1 (i.e. ≥ 50%), the nanoemulsion characteristics have an average size below 300 nm and a polydispersity index below 0.32.

For all compositions, the surface charge measured with the same instrument using phase analysis light scattering was found to be positive with zeta potential between +4 and +21 mV.

### Example 2

The purpose of this example was to describe the preparation of a dry power obtained by drying the submicron particulate adjuvant onto a solid carrier and to evaluate the particle size distribution obtained after redispersion in water of the obtained dry powder.

In these preparations, the submicron particulate adjuvant was a O/W nanoemulsion (NE) prepared using PEG 660 12-hydroxystearate as surfactant and an SOR of 50% (1:1), as described in Example 1.

Three different solid carriers for the NE were selected: calcium carbonate (Destab™ 90S, Seppic, Puteaux, France), mannitol (Pearlitol® 200 DC, Roquette Pharma, Lestrem, France) and their mixture 50:50. Particle size fraction between 38 and 106 µm was obtained for each solid carrier by sieving to obtain particles with dimensions suitable for nasal administration (Endecott Sieves, London, UK).

The liquid dispersion of submicron particulate adjuvant was deposited onto the solid carrier by means of a controlled wetting/drying process, as follows: the solid carrier powder was put inside an apparatus consisting in a thermostated bowl at 45°C and continuous mixing. The solid carrier powder was wetted progressively with small volumes of the NE using a spray pump. The powder was mixed to distribute the liquid in the powder bed and to avoid macroscopic agglomeration.

At regular intervals, typically after adding an amount of liquid in weight ratio 0.25:1 to the solid, the powder was let to dry at 45°C for 30 minutes. Vacuum (950-990 mbar depression) was applied to speed up evaporation.

Further preparations were obtained by using a liquid to solid ratio of 1:1, 1:1.5 and 2:1 (in this example being expressed by the corresponding "solid to liquid" wt. ratios of 1:1, 2:3 and 1:2, respectively).

The morphology and shape of the powders and powders with nanoemulsion were examined by Gemini scanning electron microscope (Zeiss, Hamburg, Germany). For conventional imaging in SEM, the samples were deposited on a carbon tape support and directly analyzed at 1.00 kV under vacuum.

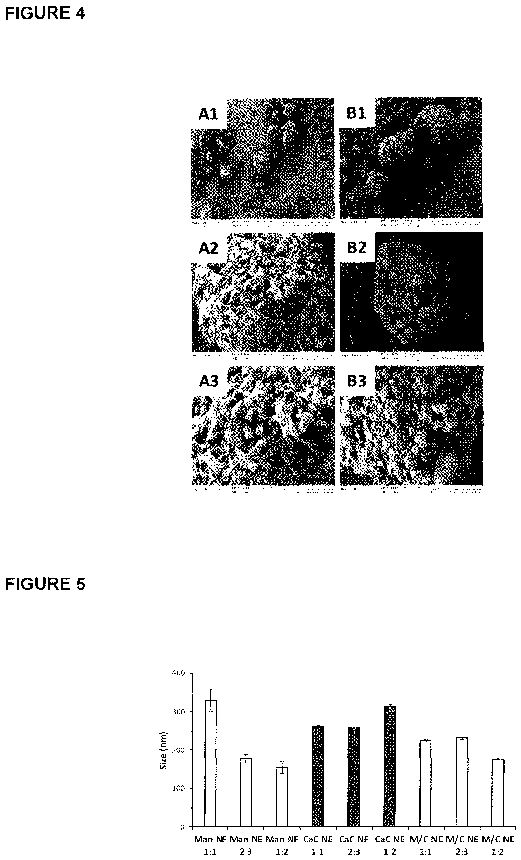

The images were obtained at different magnification factors are shown in FIG. 4: it can be appreciated the formation of relatively small agglomerates of few tens of microns and the presence on the agglomerates surface of nanometric size structures (filaments, ruggedness) not present in the starting material.

Once adsorbed on the carrier powders, the nanoemulsion acts as an adjuvant and is released as particulate with size distribution close to the original particle size upon redispersion in biological fluids. In order to asses if it was still possible to reobtain a nanoemulsion from the dry powders, they were dispersed in a fixed amount of water (0.5 g of each powder were dissolved in 10 ml ultrapure water).

After redispersion, the samples were centrifuged for 5 minutes at 2000×*g* in order to separate undissolved powder. The supernatant was then collected and further diluted with ultrapure water in proportion 1:3 for particle size analysis by dynamic light scattering (Zetasizer Nano ZS, Malvern Pananalytical, Malvern, UK).

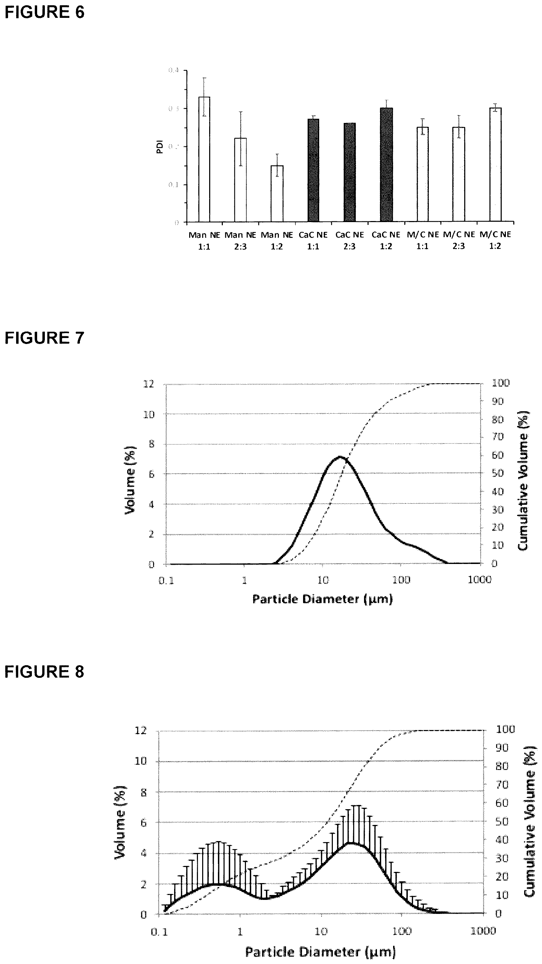

Average particle size and polydispersity index (PDI) measured are presented in FIG. 5 and FIG. 6 respectively. Particles obtained after redispersion showed average particle size ranging from 150 to 330 nm, with polydispersity index from 0.15 to 0.33. Considering that the starting nanoemulsion average particle size and PDI were 130 nm and 0.250, respectively, it appears that the drying process on the solid carrier is preserving the dimensional characteristics of the submicron particulate adjuvant.

### Example 3

The purpose of this example was to describe the preparation of a dry power vaccine obtained by drying the submicron particulate adjuvant mixed with *Mycoplasma hyopneumoniae* antigen onto a solid carrier and to evaluate the particle size distribution obtained after redispersion in water of the obtained dry powder.

In this example the submicron particulate adjuvant was an O/W nanoemulsion prepared using PEG 660 12-hydroxystearate as surfactant and an SOR of 1:1 (50%), as described in Example 1.

The mixture between inactivated whole-cell concentrate *Mycoplasma hyopneumoniae* (1*10¹⁰ bacterins/ml in water, stored at 4°C) and the nanoemulsion in volume ratio 60:40 (NE-*MHyo*) was obtained under stirring at room temperature.

In this example the solid carrier selected was mannitol (Pearlitol® 200 DC, Roquette Pharma, Lestrem, France; particle size fraction between 38 and 106 µm obtained by sieving, Endecott Sieves, London, UK).

The liquid dispersion of submicron particulate adjuvant and antigen was deposited onto the solid carrier by means of a controlled wetting- and subsequent drying process described in Example 2, using a liquid to solid ratio 2:1.

Once adsorbed on the solid carrier, the nanoemulsion and *Mycoplasma hyopneumoniae* whole cells should ideally be released in their original particle size distribution upon redispersion in biological fluids. In order to asses if it was still possible to redisperse both nanoemulsion and antigen, 0.4 g of powders were dispersed in 2 mL of ultrapure water and further diluted with 70 mL before analysis by laser diffraction performed using the wet unit system of the Spraytec instrument (Malvern Instruments Ltd, UK).

The results for the dry powder vaccine redispersion and for the inactivated whole-cell concentrate *Mycoplasma hyopneumoniae* measured for comparison are presented in FIG. 7 and 8 and show the particle size frequency distribution by volume and particle size cumulative volume distribution.

From FIG. 8, it can be appreciated that the redispersion in water of the dry powder vaccine presented in this example is providing the redispersion of two populations of particles. One population with a peak near 13 µm can be attributed to the *Mycoplasma hyopneumoniae* antigen, as demonstrated by its particle size distribution presented in FIG. 7, while the second population with a peak below 1 µm and centered around 550 nm can be attributed to the submicronic particulate antigen.

### Example 4

The purpose of this example was to demonstrate the induction of a local immune response in piglets after the nasal administration of a dry power vaccine obtained by drying the submicron particulate adjuvant mixed with *Mycoplasma hyopneumoniae* antigen onto a solid carrier according to the procedure presented in Example 3.

In this example the submicron particulate adjuvant was an O/W nanoemulsion prepared using PEG 660 12-hydroxystearate as surfactant and an SOR of 1:1 (50%) as described in Example 1.

The mixture between inactivated whole-cell concentrate *Mycoplasma hyopneumoniae* (1*10¹⁰ bacterins/ml in water, stored at 4°C) and the nanoemulsion in volume ratio 66:33 (NE-*MHyo*) was obtained under stirring at room temperature.

In this example the solid carrier selected was calcium carbonate (Destab™ 90S, Seppic, Puteaux, France; particle size fraction between 38 and 106 µm obtained by sieving, Endecott Sieves, London, UK).

The liquid dispersion of submicron particulate adjuvant and antigen was deposited onto the solid carrier by means of a controlled wetting/drying process described in Example 2, using a liquid to solid ratio 2:1

For this example, the obtained dry powder vaccine was administered to piglets (Department of Veterinary Sciences, University of Parma, Italy; Sper. Min. Aut. 519/2017-PR) with a model nasal delivery device (Monodose Nasal Insufflator, MIAT, Milano, Italy). This nasal device system works with capsules (HPMC size 3, Qualicaps, Alcobendas, Spain) in which 25 mg of dry powder vaccine was pre-dosed. Two capsules of dry powder vaccine were administered to each piglet one for each nostril. Nasal mucosa tissue samples were taken from animals sacrificed on day 2, 5 and 9 post-vaccination and within 12 hours fixed in formalin 10% and then routinely processed before being embedded in paraffin. Tissue sections of 5 µm thickness were then obtained with a Leica microtome.

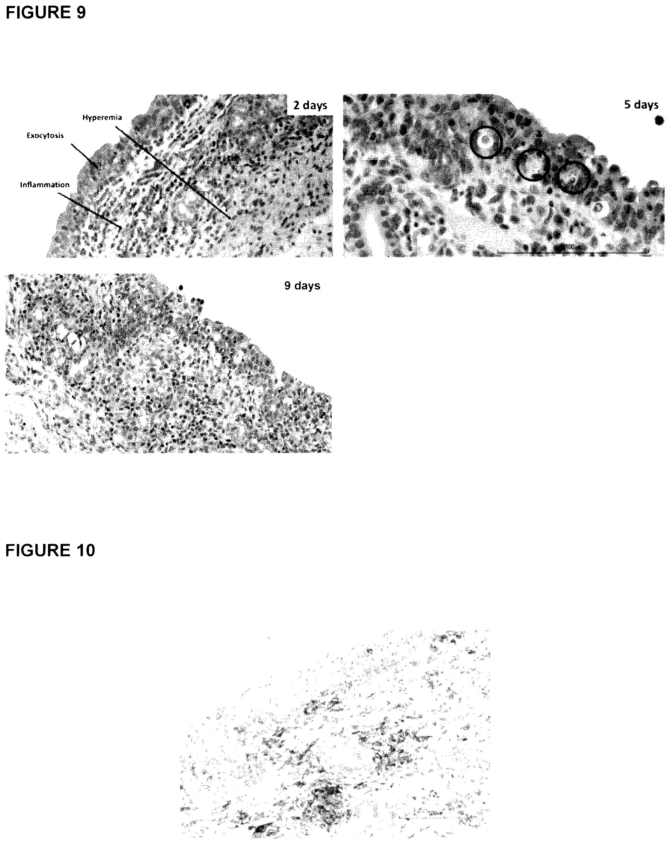

Samples for histologic examination were then stained with hematoxylin and eosin (HE) and Giemsa for subsequent examination by light microscopy (Eclipse E800, Nikon, Tokyo, Japan) (FIG. 9). At 2 and 5 days post-vaccination, nasal mucosa was characterized by hyperemia, infiltration of lymphocytes, plasma cells and macrophages with focal severe granulocyte exocytosis. Macrophages show sign of intense phagocytic activity. At day 9 post-vaccination, reduction of hyperemia and granulocyte exocytosis was evident, although deeper layer of the mucosa and submucosa were expanded by elevate amount of lymphomononuclear cells.

Some samples further underwent immunohistochemistry in order to evaluate the Swine Leukocyte Antigen (SLA) positive cells (mouse anti pig SLA class II DQ). For immunohistochemistry staining the protocol proposed by Sarradell *et al.* was followed ²⁸. Briefly Immunohistochemistry (IHC) was performed with the standard avidin-biotin-peroxidase complex (ABC) procedure with a commercial kit (Vectastain Standard Elite; Vector Laboratories, Burlingame, California, USA). Primary mono/polyclonal antibody to detect pig SLA class II DQ was used. Labelling was visualized with 3,3'-Diaminobenzidine, and sections were counterstained with Mayer's hematoxylin.

All evaluated samples of animals treated intranasally, showed numerous cell positive with an intense expression of SLA Class II antigen (i.e. lymphocytic and macrophagic cells), confirming a robust local immune response (FIG. 10).

### Example 5

The purpose of this example was to demonstrate the induction of a systemic immune response in piglets after the nasal administration of a dry power vaccine obtained by drying the submicron particulate adjuvant mixed with *Mycoplasma hyopneumoniae* antigen onto a solid carrier according to the composition presented in Example 4.

For this example, the obtained dry powder vaccine was administered to piglets (Department of Veterinary Science, University of Parma, Italy; Sper. Min. Aut. 519/2017-PR) with a model nasal delivery device (Monodose Nasal Insufflator, MIAT, Milano, Italy). This nasal device system works with capsules (HPMC size 3, Qualicaps, Alcobendas, Spain) in which 25 mg of dry powder vaccine was pre-dosed. Two capsules of dry powder vaccine were administered to each piglet one for each nostril.

Vaccine efficacy was evaluated determining the frequencies of *Mycoplasma hyopneumoniae*-specific IFN-γ secreting cells (SC) as well as the memory B cell response (CD79α+IgA+ and CD79α+IgG+ cells) in peripheral blood mononuclear cells after *ex vivo* and *in vitro* re-challenge, respectively. The results were compared with those obtained by intramuscular (IM) vaccination with 2 ml of inactivated whole-cell concentrate of *Mycoplasma hyopneumoniae.*

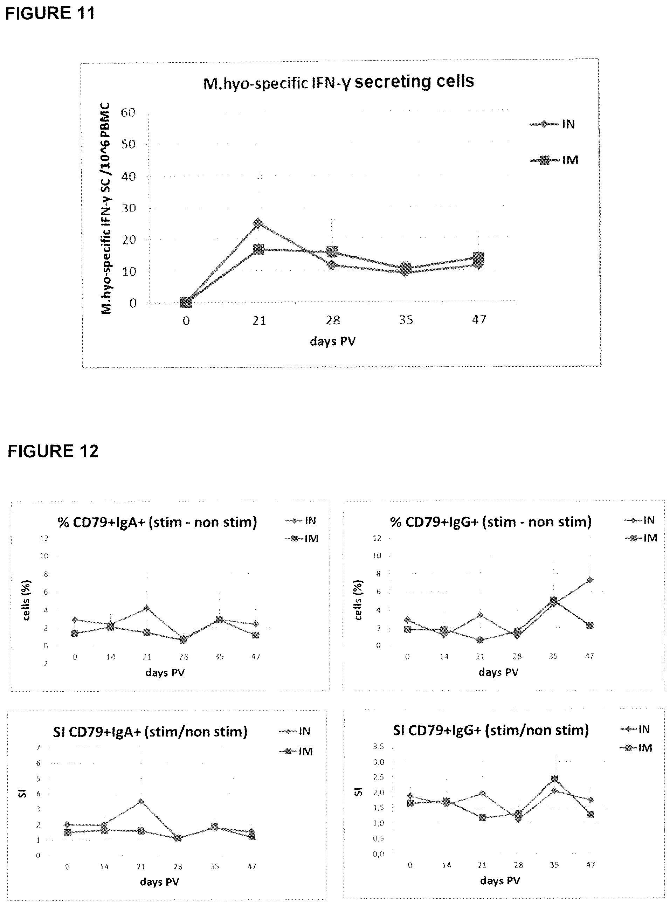

The frequencies of *Mycoplasma hyopneumoniae* (*Mhyo*)-specific IFN-γ secreting cells (SC) in pig PBMC were determined by ELISPOT as previously described²⁹ (FIG.11). PBMC were isolated by Histopaque-1077® (Sigma-Aldrich, St. Louis, MO, USA), plated at 8 × 10⁵ cells/well in complete medium (cRPMI, RPMI-1640 + 10% FBS) into 96-well plates (MultiScreen HTS-IP, Millipore, Billerica, MA, USA) coated with 10 µg/ml anti-pig IFN-γ mAb (clone P2G10, BD Pharmingen, Franklin Lakes, NJ, USA) and blocked with cRPMI.

For the *ex vivo* antigen recall, cells were stimulated with inactivated *Mhyo* strain 11 at 100 bacterins/ml (∼50 µg/ml) in cRPMI for 20 h. Plates were developed by using 0.5 µg/ml anti-pig IFN-γ biotin-labelled mAb (clone P2C11, BD Pharmingen, Franklin Lakes, NJ, USA) and 1:750 AP-conjugated anti-biotin mAb (Vector Labs, Burlingame, CA, USA). Plates were incubated with BCIP/NBT (BioRad, Hercules, CA, USA) and the reaction was stopped with distilled water. The frequencies of *Mhyo*-specific IFN-γ SC were quantified using an AID® ELISpot Reader and AID® ELISpot software v.6.0 (Autoimmun Diagnostika, Strassberg, Germany). As positive control, 4 × 10⁵ PBMC/well were incubated with the polyclonal activator PHA (10 µg/ml) while, as negative control, 8 × 10⁵ PBMC were incubated without antigen. The negative control values were subtracted from the respective counts of the stimulated cells and the immune response was expressed as number of IFN-γ SC/10⁶ PBMC (IFN-γ SC frequency).

The fraction of *Mycoplasma hyopneumoniae* (*Mhyo*)-specific IFN-γ secreting cells (SC) in pig PBMC was not significantly different when comparing the intranasal vaccination with the dry powder vaccine object of this patent and a traditional intramuscular immunization with the same antigen.

The fraction of memory B cells expressing surface IgA or IgG in the peripheral blood of IM- and IN-vaccinated pigs was quantified after *ex vivo* stimulation. Briefly, isolated PBMC were plated at 3 x 10⁶ cells/ml in 24-well plates in cRPMI-1640 + 10% FBS and stimulated for 40 h with Mhyo antigen used for ELISPOT or kept unstimulated as negative control. The negative control values were subtracted from the respective counts of the stimulated cells and the immune response was expressed as percentage of CD79α+IgA+ and CD79α+IgG+ cells and as ratio between stimulated and unstimulated cells (stimulation index, SI).

Cell number and viability were determined after the incubation period by optical microscopy and Tryphan blue. Cells were first stained with 1/4,000 LIVE/DEAD® for 30 min. to exclude dead cells from flow cytometry analysis, washed and stained with primary mouse anti-pig IgA (clone K611B4, IgG1, BioRad, Hercules, CA, USA) or anti-pig IgG (clone F007-1241, IgG1, BD Pharmingen, Franklin Lakes, NJ, USA) for 15 min. Secondary goat anti-mouse IgG₁-FITC (1070-02, Southern Biotech, Birmingham, AL,USA) was used for both primary antibodies, incubating cells for 15 min. at 4°C, in the dark.

The memory B cell response evaluated by quantifying the percentage of CD79α+IgA+ cells after *in vitro* re-stimulation with the *Mhyo* antigen increased in IN-vaccinated pigs at 3 weeks PV in correspondence with the peak of IFN-γ secreting cells while IM-vaccinated pigs did not show a significant modulation. This was confirmed also by the stimulation index.

The levels of memory CD79α+IgG+ B cells showed a comparable course with the CD79α+IgA+ cells, especially as percentages.

This confirms the efficacy of the dry powder vaccine object of this invention in terms of induction of a systemic immunity at an extent equivalent to a conventional intramuscular immunization with the same antigen.

### Bibliography

1. Pfleiderer, M. Stability of vaccines - bridging from stability data to continuous safety and efficacy throughout shelf life - an always reliable approach? Biologicals 37, 364-368 (2009).
2. Woodrow, K. A., Bennett, K. M. & Lo, D. D. Mucosal vaccine design and delivery. Annu Rev Biomed Eng 14, 17-46 (2012).
3. Csaba, N., Garcia-Fuentes, M. & Alonso, M. J. Nanoparticles for nasal vaccination. Adv Drug Deliv Rev 61, 140-157 (2009).
4. Hellings, P., Jorissen, M. & Ceuppens, J. L. The Waldeyer's ring. Acta Otorhinolaryngol Belg 54, 237-241 (2000).
5. Billich, A. Technology evaluation: Flumist, University of Michigan. Curr. Opin. Mol. Ther. 2, 340-344 (2000).
6. Kulkarni, P. S., Raut, S. K. & Dhere, R. M. A post-marketing surveillance study of a human live-virus pandemic influenza A (H1N1) vaccine (Nasovac®) in India. Hum Vaccin Immunother 9, 122-124 (2013).
7. Chadwick, S., Kriegel, C. & Amiji, M. Nanotechnology solutions for mucosal immunization. Adv Drug Deliv Rev 62, 394-407 (2010).
8. Aguilar, J. C. & Rodriguez, E. G. Vaccine adjuvants revisited. Vaccine 25, 3752-3762 (2007).
9. Levast, B. et al. Vaccine Potentiation by Combination Adjuvants. Vaccines (Basel) 2, 297-322 (2014).
10. Helgeby, A. et al. The combined CTA1-DD/ISCOM adjuvant vector promotes priming of mucosal and systemic immunity to incorporated antigens by specific targeting of B cells. J. Immunol. 176, 3697-3706 (2006).
11. Bahamondez-Canas, T. F. & Cui, Z. Intranasal immunization with dry powder vaccines. Eur J Pharm Biopharm 122, 167-175 (2018).
12. Abdelwahed, W., Degobert, G., Stainmesse, S. & Fessi, H. Freeze-drying of nanoparticles: formulation, process and storage considerations. Adv Drug Deliv Rev 58, 1688-1713 (2006).
13. Bi, R., Shao, W., Wang, Q. & Zhang, N. Spray-freeze-dried dry powder inhalation of insulin-loaded liposomes for enhanced pulmonary delivery. J Drug Target 16, 639-648 (2008).
14. Chen, K. H., Di Sabatino, M., Albertini, B., Passerini, N. & Kett, V. L. The effect of polymer coatings on physicochemical properties of spray-dried liposomes for nasal delivery of BSA. Eur J Pharm Sci 50, 312-322 (2013).
15. Huang, J. et al. A novel dry powder influenza vaccine and intranasal delivery technology: Induction of systemic and mucosal immune responses in rats. Vaccine 23, 794-801 (2004).
16. Garmise, R. J., Staats, H. F. & Hickey, A. J. Novel dry powder preparations of whole inactivated influenza virus for nasal vaccination. AAPS PharmSciTech 8, E81 (2007).
17. Jiang, G. et al. Anthrax vaccine powder formulations for nasal mucosal delivery. J Pharm Sci 95, 80-96 (2006).
18. Wang, S. H., Kirwan, S. M., Abraham, S. N., Staats, H. F. & Hickey, A. J. Stable Dry Powder Formulation for Nasal Delivery of Anthrax Vaccine. J Pharm Sci 101, 31-47 (2012).
19. Springer, M. J. et al. Preclinical dose-ranging studies of a novel dry powder norovirus vaccine formulation. Vaccine 34, 1452-1458 (2016).
20. Ball, J. P. et al. Intranasal delivery of a bivalent norovirus vaccine formulated in an in situ gelling dry powder. PLoS ONE 12, e0177310 (2017).
21. Tlaxca, J. L., Ellis, S. & Remmele, R. L. Live attenuated and inactivated viral vaccine formulation and nasal delivery: Potential and challenges. Adv Drug Deliv Rev 93, 56-78 (2015).
22. Li, X., Thakkar, S. G., Ruwona, T. B., Williams, R. O. & Cui, Z. A method of lyophilizing vaccines containing aluminum salts into a dry powder without causing particle aggregation or decreasing the immunogenicity following reconstitution. J Control Release 204, 38-50 (2015).
23. Kanojia, G. et al. Developments in the formulation and delivery of spray dried vaccines. Hum Vaccin Immunother 13, 2364-2378 (2017).
24. Velasquez, L. S. et al. Intranasal delivery of Norwalk virus-like particles formulated in an in situ gelling, dry powder vaccine. Vaccine 29, 5221-5231 (2011).
25. Saboo, S. et al. Optimized Formulation of a Thermostable Spray-Dried Virus-Like Particle Vaccine against Human Papillomavirus. Mol Pharm 13, 1646-1655 (2016).
26. Coucke, D. et al. Spray-dried powders of starch and crosslinked poly(acrylic acid) as carriers for nasal delivery of inactivated influenza vaccine. Vaccine 27, 1279-1286 (2009).
27. Trows, S. & Scherließ, R. Carrier-based dry powder formulation for nasal delivery of vaccines utilizing BSA as model drug. Powder Technology 292, 223-231 (2016).
28. Sarradell, J. et al. A morphologic and immunohistochemical study of the bronchus-associated lymphoid tissue of pigs naturally infected with Mycoplasma hyopneumoniae. Vet. Pathol. 40, 395-404 (2003).
29. Martelli, P. et al. Systemic and local immune response in pigs intradermally and intramuscularly injected with inactivated mycoplasma hyopneumoniae vaccines. Vet. Microbiol. 168, 357-364 (2014).

## Claims

1. A process to obtain a highly-active antigen aggregated formulation for mucosal administration, comprising the steps of:
(a) adding a liquid dispersion of the antigen to an oil-in-water nanoemulsion, in the respective vol/vol ratio from 10:1 to 1:1; wherein said oil-in-water nanoemulsion comprises a surfactant and an oligosaccharide or a polysaccharide, and has a surfactant/oil weight ratio (SOR) from 20% to 80%. dispersing as a spray the product of step (a) onto a solid carrier in movement, wherein said product of step (a) and said carrier are used in the respective wt/wt ratio from 0.1:1 and 4:1, and subsequently drying the resulting mixture in mild conditions.

2. The process according to claim 1, wherein the nanoemulsion used in step (a) has a water phase and an oil phase in the respective volume ratio from 1:1 to 6:1.

3. The process according to claims 1-2, wherein the nanoemulsion comprises about 5 to 30% w/w, and preferably about 20% w/w of oil phase.

4. The process according to claims 1-3, wherein the surfactant includes one or more non-ionic polyethoxylated surfactants.

5. The process according to claim 4, wherein said non-ionic polyethoxylated surfactants include PEG 660 12-hydroxystearate, polyoxyl 35 castor oil, TPGS (tocopheryl polyethyleneglycol succinate) and mixtures thereof.

6. The process according to claims 1-5, wherein said oligosaccharide or polysaccharide includes chitosan, hyaluronan, starch, inulin, dextrans, maltodextrins, cyclodextrins, and mixtures thereof.

7. The process according to claims 1-6, wherein said solid carrier includes mannitol, sorbitol, dextrose, sucrose, lactose, maltodextrin, water-soluble cellulose derivatives, calcium carbonate, calcium phosphate, microcrystalline cellulose, ethylcellulose, and mixtures thereof.

8. The process according to claims 1-7, wherein said dispersing as a spray and drying step (b) is performed under mild heating and/or vacuum conditions.

9. The process according to claims 1-8, wherein said mild conditions is a heating performed at a temperature from 30 to 50°C.

10. The process according to claims 1-9, wherein the whole volume of the product of step a) is sprayed step-wise, via a number of repeated cycles b) and c).

11. Antigen aggregated formulation obtained by the process of claims 1-10.

12. Antigen aggregated formulation according to claim 11, provided to a patient as a dry powder vaccine.

13. Antigen aggregated formulation according to claims 11-12, suitable for mucosal, preferably nasal, administration.

14. Antigen aggregated formulation according to claims 11-12, for use in a method of immunostimulation in a patient or in an animal in need thereof.

15. Antigen aggregated formulation for the use of claim 14, for administration to a mucosa, preferably a nasal mucosa.
